(19) Europäisches Patentamt / European Patent Office / Office européen des brevets

(11) EP 3 730 053 A1

(12) **EUROPEAN PATENT APPLICATION**
published in accordance with Art. 153(4) EPC

(43) Date of publication:
28.10.2020 Bulletin 2020/44

(21) Application number: 17935512.8

(22) Date of filing: 20.12.2017

(51) Int Cl.:
*A61B 5/1455* (2006.01)

(86) International application number:
PCT/JP2017/045667

(87) International publication number:
WO 2019/123559 (27.06.2019 Gazette 2019/26)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
MA MD TN

(71) Applicant: Medical Photonics Co., Ltd.
Sapporo-shi, Hokkaido 001-0021 (JP)

(72) Inventor: IINAGA, Kazuya
Sapporo-shi, Hokkaido 001-0021 (JP)

(74) Representative: Vossius & Partner
Patentanwälte Rechtsanwälte mbB
Siebertstrasse 3
81675 München (DE)

(54) **LIPID MEASUREMENT DEVICE AND METHOD THEREFOR**

(57) [Problem] To provide a device and a method that enable identification of a suitable site for lipid measurement.

[Solution] This lipid measurement device has: an irradiation unit for irradiating a predetermined site on a living body with light directed from the outside to the inside of the living body at a predetermined light intensity; a light intensity detection unit which is either spaced apart by a predetermined distance or continuous with the light irradiation position used by the irradiation unit, and detects the intensity of light released from the living body; and a control unit for calculating an in vivo static parameter on the basis of the light intensity detected by the light intensity detection unit, calculating a dynamic parameter which is an index of blood movement on the basis of a change in the static parameter over time, and determining a site on the living body which is suitable for lipid measurement on the basis of the static parameter and the dynamic parameter.

FIG. 1

EP 3 730 053 A1

## Description

Technical Field

The present invention relates to a lipid measurement device and a method therefor.

Background Art

Attention has been directed to postprandial hyperlipidemia as a risk factor for arteriosclerosis. There has been a report stating that an increase in the concentration of neutral lipid in a non-hunger state increases the risk of development of an event of coronary artery disease.

To diagnose postprandial hyperlipidemia, it is necessary to observe a change in in-blood lipid concentration for 6 to 8 hours after meals. That is, to measure the state of postprandial hyperlipidemia, it is necessary to place a subject under restraint for 6 to 8 hours and collect blood multiple times. The diagnosis of postprandial hyperlipidemia is therefore no better than clinical studies, and diagnosing postprandial hyperlipidemia at a clinical site is not practical.

Patent Literature 1 discloses an approach to a solution of the problem described above. According to the approach disclosed in Patent Literature 1, noninvasive lipid measurement can eliminate blood collection. The in-blood lipid can therefore be measured not only in a medical institution but at home. Allowing instantaneous data acquisition allows temporally continuous in-blood lipid measurement.

Citation List

Patent Literature

Patent Literature 1: International Publication No. 2014/087825

Summary of Invention

Technical Problem

In an approach for calculating the lipid concentration based on an optical scatter coefficient, the result of the approach depends on the measurement site, that is, there are sites where a change in lipid is readily measured and sites where it is difficult to measure a change in lipid. Further, these sites vary on an individual basis. It is therefore necessary to search for a measurement site appropriate for a patient by performing the measurement multiple times. The situation described above is nothing else but measurement conditions, such as the skin color, the depth of the blood layer, the thickness of a blood vessel, and other factors, which vary on an individual basis.

The present invention has been made to solve the problem with the related art, and an object of the present invention is to provide a device and a method capable of identifying a site suitable for lipid measurement.

Solution to Problem

A lipid measurement device according to the present invention includes a radiation unit that radiates light having a predetermined optical intensity to a predetermined site of a living body from a point outside the living body toward an interior of the living body, at least one optical intensity detection unit that is spaced apart by a predetermined gap or continuously from a position irradiated with the light from the radiation unit and detects optical intensity of light emitted from the living body, and a control unit that calculates a static parameter of the light in the living body based on the optical intensity detected by the optical intensity detection unit, calculates a dynamic parameter based on a temporal change in the static parameter, and determines a living body site appropriate for lipid measurement based on the static parameter and the dynamic parameter.

A lipid measurement method according to the present invention causes a computer of a lipid measurement device including a radiation unit that radiates light having a predetermined optical intensity to a predetermined site of a living body from a point outside the living body toward an interior of the living body and at least one optical intensity detection unit that is spaced apart by a predetermined gap or continuously from a position irradiated with the light from the radiation unit and detects optical intensity of light emitted from the living body to carry out the processes of calculating a static parameter of the light in the living body based on the optical intensity detected by the optical intensity detection unit, calculating a dynamic parameter based on a temporal change in the static parameter, and determining a living body site appropriate for lipid measurement based on the static parameter and the dynamic parameter.

A lipid measurement device according to the present invention is a lipid measurement device connected to a user device including a radiation unit that radiates light having a predetermined optical intensity to a predetermined site of a living body from a point outside the living body toward an interior of the living body and at least one optical intensity detection unit that is spaced apart by a predetermined gap or continuously from a position irradiated with the light from the radiation unit and detects optical intensity of light emitted from the living body, the lipid measurement device including a control unit that calculates a static parameter of the light in the living body based on the optical intensity detected by the optical intensity detection unit, calculates a dynamic parameter based on a temporal change in the static parameter, and determines a living body site appropriate for lipid measurement based on the static parameter and the dynamic parameter.

Advantageous Effects of Invention

The lipid measurement device and method according to the present invention allow identification of a site suitable for lipid measurement.

Brief Description of Drawings

[Figure 1] Figure 1 shows the configuration of a lipid measurement device according to an embodiment.
[Figure 2] Figure 2 shows that light is scattered by lipid in the blood.
[Figure 3] Figure 3 shows that light is scattered by lipid in the blood.
[Figure 4] Figure 4 shows the configuration of a control system of the lipid measurement device according to the embodiment.
[Figure 5] Figure 5 is a flowchart of a lipid measurement process in the embodiment.
[Figure 6] Figure 6 shows the configuration of a lipid measurement system according to an embodiment.
[Figure 7] Figure 7 shows the configuration of a control system of a lipid measurement device according to the embodiment.
[Figure 8] Figure 8 is a flowchart of a lipid measurement process in the embodiment.
[Figure 9] Figure 9 shows a result of a lipid loading test.
[Figure 10] Figure 10 shows optical paths in optical measurement.
[Figure 11] Figure 11 shows preferable ranges of a scatter coefficient $\mu s'$ and a variation coefficient CV.
[Figure 12] Figure 12 shows results of the measurement made on sites of right and left hands where conditions are satisfied.
[Figure 13] Figure 13 shows preferable ranges of an average blood flow rate and a variation coefficient of the blood flow rate.

Description of Embodiment

A lipid measurement device and a method therefor that are an embodiment of the present invention will be described below in detail with reference to the drawings.

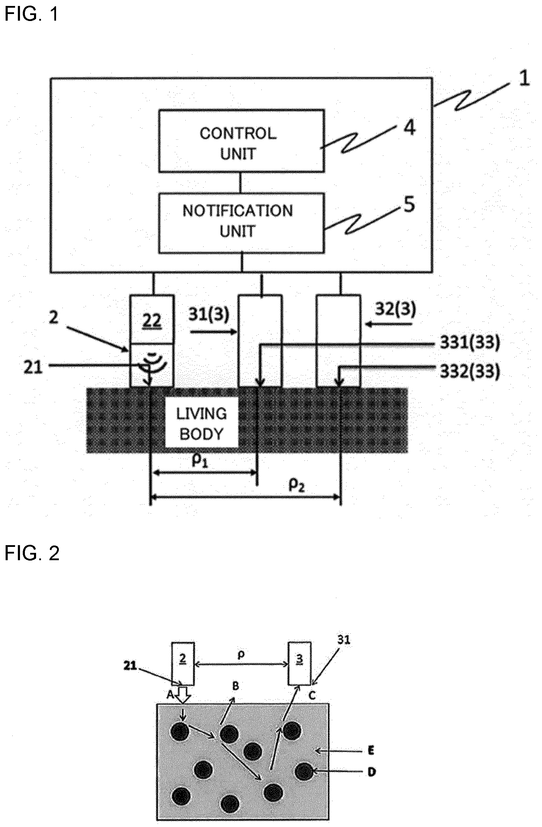

Figure 1 shows the configuration of the lipid measurement device according to the embodiment.

As shown in Figure 1, a lipid measurement device 1 according to the embodiment includes a radiation unit 2, an optical intensity detection unit 3, a control unit 4, and a notification unit 5, as shown in Figure 1.

The radiation unit 2 includes a light source 22 for radiating light to a predetermined radiation position 21 on the predetermined site of a living body from a point outside the living body toward the interior of the living body. The light source 22 can adjust the wavelength of the radiated light. The light source 22 can adjust the range of the wavelength in such a way that the wavelength range does not fall within the range of the wavelengths at which the light is absorbed by inorganic substances of the blood plasma. The light source 22 can perform the adjustment in such a way that the wavelength range does not fall within the range of the wavelengths at which the light is absorbed by the cell components of the blood. The cell components of the blood used herein are the red blood cells, white blood cells, and platelets in the blood. The inorganic substances of the blood plasma are water and electrolytes in the blood.

The range of the wavelength of the light radiated by the light source 22 is preferably formed of the range shorter than or equal to about 1400 nm and the range from about 1500 to 1860 nm in consideration of the range of the wavelengths at which the light is absorbed by the inorganic substances of the blood plasma. Further, the range of the wavelength of the light radiated by the light source 22 is more preferably formed of the range from about 580 to 1400 nm and the range from about 1500 to 1860 nm in consideration of the range of the wavelengths at which the light is absorbed by the cell components of the blood.

The thus set wavelength range employed by the light source 22 suppresses the influence of the light absorption made by the inorganic substances of the blood plasma and the influence of the light absorption made by the cell components of the blood on the light to be detected by the optical intensity detection unit 3, which will be described later. In the thus set wavelength range, no absorption large enough to identify a substance is present, whereby optical energy loss due to the absorption is negligibly small. The light in the blood therefore propagates over a large distance when scattered by lipid in the blood and exits out of the living body.

The radiation unit 2 in the embodiment can arbitrarily adjust the time length of light radiation, for example, continuous light radiation or pulsed light radiation in accordance with a method for calculating a scatter coefficient $\mu s'$ calculated by the control unit 4, which will be described later. The radiation unit 2 can arbitrarily modulate the intensity or phase of the radiated light.

The radiation unit 2 may include a light source 22 having a fixed wavelength. The radiation unit 2 may include the combination of a plurality of light sources that output light fluxes having different wavelengths or may output the combination of light fluxes having a plurality of wavelengths. The radiation unit 2 is, for example, a fluorescent lamp, an LED, a laser, an incandescent lamp, an HID, or a halogen lamp. The illuminance of the light from the radiation unit 2 may be controlled by the control unit 4 or a separately provided control circuit.

The optical intensity detection unit 3 receives light emitted from the living body toward the space outside the living body and detects the optical intensity of the light. In a case where a plurality of optical intensity detection units 3 are used, the optical intensity detection units 3 are disposed at different distances from the radiation position 21 as a rough center. In the embodiment, a first optical intensity detection unit 31 and a second optical intensity detection unit 32 are sequentially arranged from the radiation position 21 along a straight line at a predetermined interval in a single plane, as shown in Figure 1. The optical intensity detection unit 3 may be formed of a photodiode, a CCD, or a CMOS device.

In the embodiment, let $\rho 1$ be a first radiation detection distance from the radiation position 21 to a first detection position 331, where the first optical intensity detection unit 31 detects light, and let $\rho 2$ be a second radiation detection distance from the radiation position 21 to a second detection position 332, where the second optical intensity detection unit 32 detects light, as shown in Figure 1.

A predetermined distance $\rho$ is set between the radiation position 21, where the living body is irradiated with the light, and a detection position 31, where the intensity of the light emitted from the blood (E in Figure 2) in the living body is detected, as shown in Figure 2. The thus set predetermined distance $\rho$ suppresses the influence of the light directly emitted from the living body (B in Figure 2), which is the radiated light (A in Figure 2) reflected off the surface of the living body and scatterers in the vicinity of the surface. The radiated light reaches the depth where lipid, such as lipoprotein, is present and is then reflected off the lipid (D in Figure 2) in the blood. After the radiated light is reflectively scattered by the lipid, the optical intensity of the resultant backscattered light (C in Figure 2) emitted from the living body is detected. Increasing the distance $\rho$ between the radiation position 21 and the detection position 31 increases the optical path length. The number of times when the light collides with the lipid therefore increases, so that the detected light is greatly affected by the scattering. Increasing the distance $\rho$ allows the influence of the scattering that is small and has therefore been difficult to detect in related art to be readily captured.

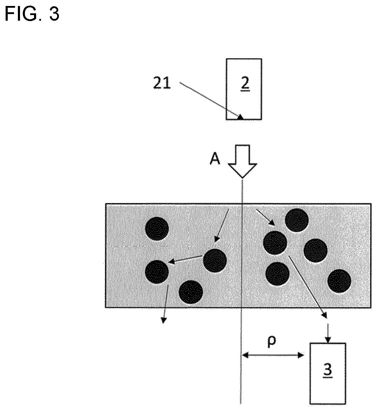

Instead, the living body (E in Figure 2) may be sandwiched between the radiation unit 2 and the optical intensity detection unit 3, and the optical intensity detection unit 3 may detect the light from the radiation unit 2, as shown in Figure 3.

Lipoprotein, which is the target under measurement, has a spherical structure covered with apoprotein and other substances. Lipoprotein is present in the form of a solid-like state in the blood. Lipoprotein is characterized in that it reflects light. In particular, chylomicrons (CM), VLDL, and other substances having a large particle diameter and specific gravity contain a large amount of triglyceride (TG) and are characterized in that they are more likely to scatter light. The optical intensity detected by the optical intensity detection unit 3 is therefore affected by the light scattered by lipoprotein.

In the case where a plurality of detection positions 31 are set, the detection positions 31 are not necessarily arranged linearly as long as they are arranged at different distances from the radiation position 21 as a rough center, and a circular arrangement, a wavy arrangement, a zigzag arrangement, or any other arrangement can be selected as appropriate. The first radiation detection distance $\rho 1$ and the second radiation detection distance $\rho 2$ from the radiation position 21 to the detection position 31 and the gap between the detection positions 331 and 332 are not limited to a fixed value and may instead be continuously changed.

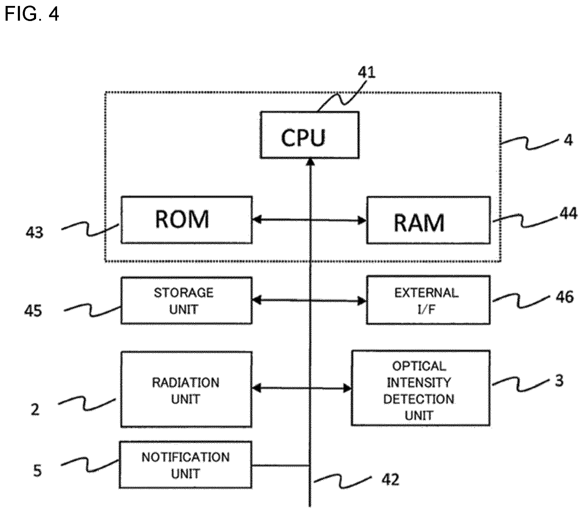

The configuration of a control system of the lipid measurement device 1 will next be described. Figure 4 is a block diagram of the lipid measurement device 1 according to the embodiment. A CPU (central processing unit) 41, a ROM (read only memory) 43, a RAM (random access memory) 44, a storage unit 45, an external I/F (interface) 46, the radiation unit 2, the optical intensity detection unit 3, and the notification unit 5 are connected to each other via a system bus 42. The CPU 41, the ROM 43, and the RAM 44 form the control unit (controller) 4.

The ROM 43 stores in advance a program executed by the CPU 41 and thresholds used by the CPU 41.

The RAM 44 has an area where the program executed by the CPU 41 is developed, a variety of memory areas, such as a work area where the program processes data, and other areas.

The storage unit 45 stores data prepared in advance on appropriate numerical ranges of static parameter and dynamic parameter. The storage unit 45 may be an internal memory that stores information in a nonvolatile manner, such as an HDD (hard disk drive), a flash memory, and an SSD (solid-state drive).

The external I/F 46 is an interface for communication with an external device, for example, a client terminal (PC). The external I/F 46 only needs to be an interface that performs data communication with an external device and may, for example, be an instrument (such as USB memory) locally connected to the external device or a network interface for the communication via a network.

The control unit 4 calculates the static parameter in the living body based on the optical intensity detected by the optical intensity detection unit 3. The optical intensity detected by the optical intensity detection unit 3 is affected by the light scattered by lipoprotein, as described above. The scatter coefficient $\mu$s' is calculated based on the fact described above. The static parameter in the embodiment is not limited to typical quantified efficiency of the scattering process and also includes scattering influence quantified under a fixed condition in consideration of a scattering phenomenon. A detailed description of the static parameter will be made below.

The control unit 4 in the embodiment calculates an optical intensity ratio or an optical intensity difference, as shown in Figure 1.

The control unit 4 calculates the scatter coefficient $\mu$s' from the ratio among the optical intensities detected in a plurality of positions by the optical intensity detection unit 3. The control unit 4 calculates the scatter coefficient μs' based on a scattering phenomenon in which the amount of attenuation of the radiated light due to the scattering increases with the distance to a detection position 33.

The radiation unit 2 radiates continuous light having a predetermined optical intensity, and the control unit 4 calculates the scatter coefficient μs' from the ratio between a first optical intensity R (ρ1) detected by the first optical intensity detection unit 31 and a second optical intensity R (ρ2) detected by the second optical intensity detection unit 32 (Expression 1).

$$(Expression\ 1)$$

$$μs'=R(ρ1)/R(ρ2)$$

The control unit 4 calculates the scatter coefficient μs' from the difference in optical intensity between the plurality of positions detected by the optical intensity detection unit 3. The control unit 4 calculates the scatter coefficient μs' based on the scattering phenomenon in which the amount of attenuation of the radiated light due to the scattering increases with the distance to the detection position 33.

The control unit 4 calculates the scatter coefficient μs' from the difference in optical intensity between the optical intensity R (ρ1) in the first detection position 331 and the optical intensity R (ρ2) in the second detection position 332 (Expression 2).

$$(Expression\ 2)$$

$$μs'=R(ρ1)-R(ρ2)$$

The method for calculating the scatter coefficient μs' calculated by the control unit 4 is not limited to the calculation methods described above.

The control unit 4 calculates the dynamic parameter, which is an index for analyzing the motion of the blood by using a standard deviation, Brownian motion, an autocorrelation function, frequency analysis, speckles, Doppler shift, Reynold's number, blood flow rate, the amount of blood, pulsation width, and other factors to measure the motion of the blood. The dynamic parameter is an index of the motion of the blood. The control unit 4 may calculate the dynamic parameter by using the amount of change in the optical intensity over an optical intensity measurement period shorter than or equal to 20 sec.

To measure a measurement target site in related art, attention is not directed to the amount of change with time in measured value, but the average of the changes is employed. In the blood measurement, however, measurement of a site where the blood is abundant or dense, such as a vein, allows a large number of pieces of information on the blood to be provided, whereby the number of noise factors decreases. In the noninvasive measurement, to evaluate whether incident light has passed through a vein, it is desirable to acquire information provided from the blood flow.

To measure heart beat periodicity, such as the pulse, it is believed that an artery is desirably used. Therefore, to position the lipid measurement device in a case where a vein is the target under measurement, it is desirable to measure variation in temporal change due to the blood flow in the received optical intensity for a fixed period.

That is, when a pulsation period (from about 0.5 to 2.0 Hz) is observed, it can be said that the skin layer is a living body site appropriate for the lipid measurement. On the other hand, a non-periodic dynamic parameter showing no pulsation period is information representing the position of a vein (at least depending on vein information), and it can therefore be said that the veins are is a living body site appropriate for the lipid measurement.

To distinguish the two types of information described above from each other, the sampling rate employed by a light receiver is desirably 10 msec or shorter, and the resolution of the light receiver is desirably at least 16 bits.

The dynamic parameter includes a variation coefficient CV of the scatter coefficient μs'. The control unit 4 calculates the variation coefficient CV of the scatter coefficient μs' based on a temporal change in the calculated scatter coefficient μs'. The variation coefficient CV can be calculated, for example, by Expression 1 below.

$$[Expression\ 1]$$

$$X = \frac{1}{T\bar{x}} \int_0^T x\, d\theta$$

T : Measurement period
x : Measured optical intensity
$\bar{x}$ : Average of measured optical intensities
θ : Period
X : Disturbance in fixed period = variation coefficient CV of scatter coefficient μs'

To calculate the variation coefficient CV, the period for which the scatter coefficient μs' is measured may be longer than or equal to 1 msec but shorter than or equal to 30 sec, preferably, longer than or equal to 5 msec but shorter than or equal to 25 sec, more preferably longer than or equal to 10 msec but shorter than or equal to 20 sec ("sec" is abbreviation for "second").

The control unit 4 calculates the static parameter and the dynamic parameter to determine a site appropriate for the lipid measurement based on the calculated static parameter and the calculated dynamic parameter.

The blood that is the target under measurement flows through a blood vessel, unlike, for example, the skin tissue. In the embodiment, the dynamic parameter is calculated by measurement for a fixed period in preparation for the analysis. Further, the static parameter representing the entire scattering present in the optical path is caluculated. An optimum site for the lipid measurement for each individual is then determined from the two parameters.

The static parameter and the dynamic parameter are not necessarily acquired via a communication line and may be manually input.

The storage unit 45 in the embodiment stores data prepared in advance on an appropriate numerical range of each of the static parameter and the dynamic parameter. The control unit 4 compares the data stored in the storage unit 45 with the calculated static parameter and the calculated dynamic parameter to evaluate whether or not the predetermined site is a site appropriate for the lipid measurement.

The control unit 4 determines that the predetermined site is a site appropriate for the lipid measurement when the scatter coefficient μs' is greater than or equal to 0.4 but smaller than or equal to 0.53 and the variation coefficient CV is greater than or equal to 0.1% but smaller than or equal to 5.0%, preferably, the scatter coefficient μs' is greater than or equal to 0.41 but smaller than or equal to 0.51 and the variation coefficient CV is greater than or equal to 0.2% but smaller than or equal to 1.5%, more preferably, the scatter coefficient μs' is greater than or equal to 0.42 but smaller than or equal to 0.46 and the variation coefficient CV is greater than or equal to 0.5% but smaller than or equal to 1.0%. The reason why the numerical range described above is employed will be described in the section of Example. The method using the variation coefficient CV allows a simple device configuration and simple calculation and is therefore excellent as a simple approach.

In the above description, the static parameter is the scatter coefficient, and the dynamic parameter is the variation coefficient of the scatter coefficient. Instead, the static parameter can be an average blood flow rate for a fixed period ("an average blood flow rate for a fixed period" is hereinafter also referred to as "an average blood flow rate"), and the dynamic parameter can be the variation coefficient of the average blood flow rate for the fixed period ("the variation coefficient of the average blood flow rate for the fixed period" is hereinafter also referred to as "the variation coefficient of the blood flow rate").

The control unit 4 calculates the static parameter (average blood flow rate) in the living body based on the optical intensity detected by the optical intensity detection unit 3. To detect the amount of blood, the number of light receivers can be one, and the distance between the light incident position and the light receiver can be zero.

The control unit 4 in the embodiment calculates the average blood flow rate based on the optical intensity detected by the optical intensity detection unit 3. The blood flow rate may be measured by using Doppler shift or speckles. The principle in accordance with which the blood flow rate is measured is the same as that employed by a typical laser blood flow meter, and the blood flow rate is measured by measuring the difference in phase between received optical components that occurs when the scatterers move under light radiation (an example of the measurement principle is described in the following URL: http://www.omegawave.co.jp/products/flo/principle.shtml). A temporal change in the phase difference or any other parameter represents the blood flow rate. In the embodiment, the static parameter is the value obtained by averaging the blood flow rates over a specific temporal range (that is, average blood flow rate).

The control unit 4 calculates the dynamic parameter (variation coefficient of amount of blood), which is an index for measuring the motion of the blood, from the static parameter (average blood flow rate).

The dynamic parameter includes a variation coefficient of the blood flow rate for a fixed period. The control unit 4 calculates the variation coefficient of the blood flow rate from a temporal change in the average of the calculated amounts of blood. The variation coefficient of the amounts of blood can be calculated, for example, by Expression 2 below.

[Expression 2]

$$X = \frac{1}{T\bar{x}} \int_0^T x\, d\theta$$

T : Measurement period

x : Blood flow rate measured from change in intensity of received light

$\bar{x}$ : Average blood flow rate measured from change in intensity of received light

θ : Period

X : Disturbance in fixed period = variation coefficient CV of blood flow rate for fixed period

To calculate the variation coefficient of the blood flow rate, the period for which the blood flow rate is measured may be longer than or equal to 0.5 sec but shorter than or equal to 10 sec, preferably, longer than or equal to 1 sec but shorter than or equal to 5 sec.

The control unit 4 calculates the static parameter and the dynamic parameter and determines a site appropriate for the lipid measurement based on the static parameter and the dynamic parameter.

The storage unit 45 in the embodiment stores data prepared in advance on an appropriate numerical range of each of the static parameter and the dynamic parameter. The control unit 4 compares the data stored in the storage unit 45 with the calculated static parameter and the calculated dynamic parameters to evaluate whether or not the predetermined site is a site appropriate for the lipid measurement.

The control unit 4 determines that the predetermined site is a site appropriate for the lipid measurement when the average blood flow rate is greater than or equal to 3.1 mL/min but smaller than or equal to 21.0 mL/min and the variation coefficient of the blood flow rate is greater than or equal to 5% but smaller than or equal to 50%, preferably, the average blood flow rate is greater than or equal to 5.1 mL/min but smaller than or equal to 15.0 mL/min and the variation coefficient of the blood flow rate is greater than or equal to 15% but smaller than or equal to 40%, more preferably, the average blood flow rate is greater than or equal to 5.1 mL/min but smaller than or equal to 13.0 mL/min and the variation coefficient of the blood flow rate is greater than or equal to 10% but smaller than or equal to 30% ("min" is abbreviation for "minute"). The reason why the numerical range described above is employed will be described in the section of Example.

The notification unit 5 in the embodiment is, for example, a buzzer, a vibrator, or a lamp. When the control unit 4 determines that the predetermined site is a site appropriate for the lipid measurement, the control unit 4 causes the notification unit 5 to cause a buzzer to issue sound, a vibrator to vibrate, or a lamp to emit light. The notification unit 5 notifies a user that the predetermined site is a site appropriate for the lipid measurement.

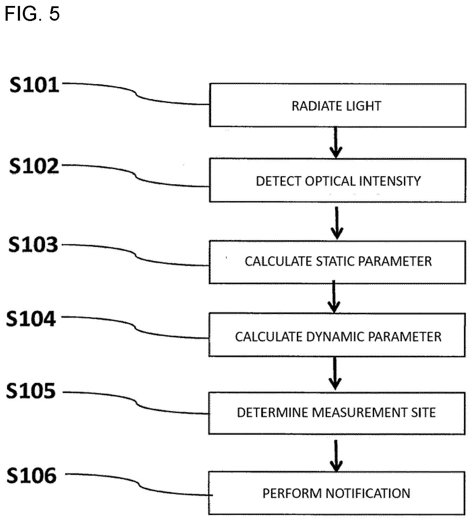

The lipid measurement device 1 having the configuration described above carries out a lipid measurement process based on a program set in advance. Figure 5 is a flowchart of the lipid measurement process in the embodiment.

The radiation unit 2 radiates continuous light to the radiation position 21 (step 101).

The first optical intensity detection unit 31 detects the optical intensity in the first detection position 331, and the second optical intensity detection unit 32 detects the optical intensity in the second detection position 332 (step 102).

The control unit 4 calculates the optical intensity difference or the optical intensity ratio between the first optical intensity in the first detection position 331 and the second optical intensity in the second detection position 332 and calculates the static parameter (scatter coefficient $\mu_s$') based on the optical intensity difference or the optical intensity ratio. Instead, the control unit 4 calculates the static parameter (average blood flow rate) from the optical intensity in the first detection position 331 or the optical intensity in the second detection position 332 (step 103).

The control unit 4 calculates the dynamic parameter, which is an index of the blood flow, from a temporal change in the static parameter (step 104). The control unit 4 may set the optical intensity measurement period to be 20 sec or shorter and calculate the dynamic parameter from the amount of change in the optical intensity within the measurement period.

The control unit 4 determines that the predetermined site of the living body irradiated with the light as a site appropriate for the lipid measurement based on the static parameter and the dynamic parameter (step 105). For example, the control unit 4 compares the data prepared in advance and stored in the storage unit 45 on the appropriate numerical range of each of the static parameter and the dynamic parameter with the calculated static parameter and the calculated dynamic parameters to evaluate whether or not the predetermined site is a site appropriate for the lipid measurement.

In the case where the static parameter is the scatter coefficient $\mu_s$' and the dynamic parameter is the variation coefficient CV, the control unit 4 determines that the predetermined site is a site appropriate for the lipid measurement when the scatter coefficient $\mu_s$' is greater than or equal to 0.4 but smaller than or equal to 0.53 and the variation coefficient CV is greater than or equal to 0.1% but smaller than or equal to 5.0%, preferably, the scatter coefficient $\mu_s$' is greater than or equal to 0.41 but smaller than or equal to 0.51 and the variation coefficient CV is greater than or equal to 0.2% but smaller than or equal to 1.5%, more preferably, the scatter coefficient $\mu_s$' is greater than or equal to 0.42 but smaller than or equal to 0.46 and the variation coefficient CV is greater than or equal to 0.5% but smaller than or equal to 1.0%.

In the case where the static parameter is the average blood flow rate and the dynamic parameter is the variation coefficient of the blood flow rate, the control unit 4 determines that the predetermined site is a site appropriate for the lipid measurement when the average blood flow rate is greater than or equal to 3.1 mL/min but smaller than or equal to 21.0 mL/min and the variation coefficient of the blood flow rate is greater than or equal to 5% but smaller than or equal to 50%, preferably, the average blood flow rate is greater than or equal to 5.1 mL/min but smaller than or equal to 15.0 mL/min and the variation coefficient of the blood flow rate is greater than or equal to 15% but smaller than or equal to 40%, more preferably, the average blood flow rate is greater than or equal to 5.1 mL/min but smaller than or equal to 13.0 mL/min and the variation coefficient of the blood flow rate is greater than or equal to 10% but smaller than or equal to 30%.

When the control unit 4 determines that the predetermined site is a site appropriate for the lipid measurement, the control unit 4 causes the notification unit 5 to cause a buzzer to issue sound, a vibrator to vibrate, or a lamp to emit light (step 106).

As described above, the lipid measurement device and the method for operating the same according to the present embodiment can evaluate whether or not the predetermined site is a site appropriate for the lipid measurement based on the static and dynamic parameters.

A lipid measurement device according to another embodiment will next be described. Some portions of the configuration of the lipid measurement device according to the other embodiment are the same as those of the configuration of the lipid measurement device according to the embodiment described above, and different portions will therefore be primarily described.

In the embodiment described above, the configuration in which the radiation unit 2, the optical intensity detection unit 3, the control unit 4, and the notification unit 5 are integrated with one another has been presented by way of example, but this configuration is not limiting. The radiation unit 2, the optical intensity detection unit 3, the control unit 4, and the notification unit 5 may be configured as a system in which the radiation unit 2, which radiates light, the optical intensity detection unit 3, and the notification unit 5 are configured as a user device and the control unit 4 is provided in a server device connected to the user device.

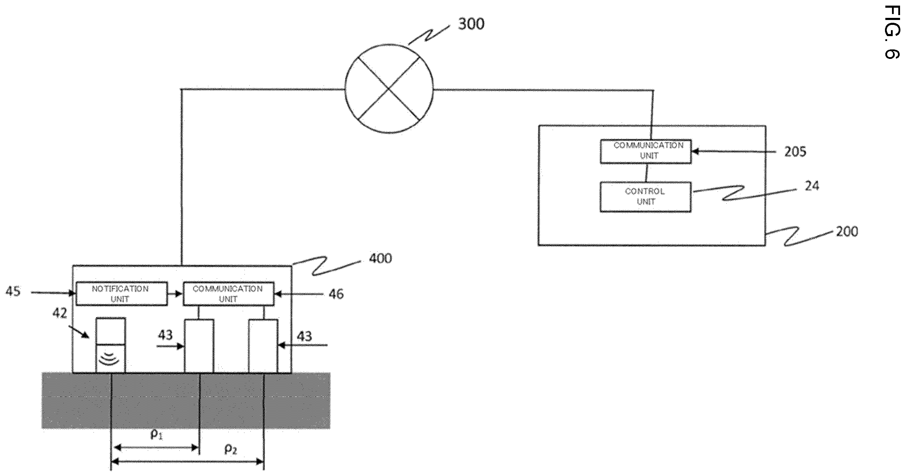

Figure 6 shows a system configuration diagram in the embodiment. The system includes a lipid measurement device 200, an access point 300, and a user device 400.

The user device 400 includes a radiation unit 42, optical intensity detection units 43, a control unit 44, a notification unit 45, and a communication unit (external I/F) 46. The configurations and functions of the radiation unit 42, the optical intensity detections unit 43, and the notification unit 45 are the same as those in the embodiment described above and will not therefore be described.

The lipid measurement device 200 according to the embodiment is communicably connected to the user device 400 via the access point 300 or any other component. A control unit 24 of the lipid measurement device 200 calculates the static parameter and the dynamic parameters from the optical intensity transmitted from the user device 400 and determines a site appropriate for the lipid measurement. The content of a specific process carried out by the control unit 24 is the same as that in the lipid measurement device 100 according to the embodiment and will therefore not be described.

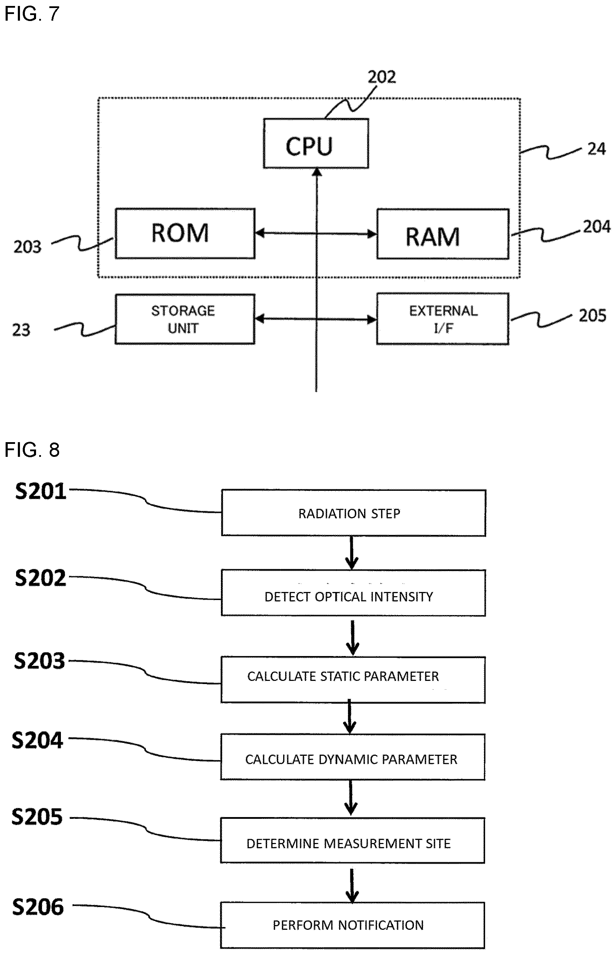

The lipid measurement device 200 according to the embodiment is, for example, a server device. The configuration of a control system of the lipid measurement device 200 according to the embodiment will be described. Figure 7 is a block diagram of the lipid measurement device 200 according to the embodiment. A CPU 202, a ROM (read only memory) 203, a RAM (random access memory) 204, a communication unit (external I/F (interface)) 205, and a storage unit 23 are connected to each other via a system bus 208. The CPU 202, the ROM 203, and the RAM 204 form the control unit 24.

The ROM 203 stores in advance a program executed by the CPU 202 and thresholds used by the CPU 202.

In the RAM 204 are dynamically formed an area where the program executed by the CPU 202 is developed, a variety of memory areas, such as a work area where the program processes data, and other areas.

The storage unit 23 stores data prepared in advance on an appropriate numerical range of each of the static parameter and the dynamic parameter. The storage unit 23 may be a device that stores information in a nonvolatile manner and is an internal storage, such as an SSD (solid-state drive) and an HDD (hard disk drive).

In the embodiment, the data is stored in the storage unit 23 and may instead be stored in the RAM 204.

The control unit 24 calculates the static parameter and the dynamic parameter from the optical intensity detected by the plurality of optical intensity detection units 43. The control unit 24 evaluates based on the static parameter and the dynamic parameter whether or not the predetermined site is a site appropriate for the lipid measurement.

The communication unit (external I/F) 205 is an interface for communication with an external device. The communication unit (external I/F) 205 only needs to be an interface that performs data communication with an external device. The communication unit (external I/F) 205 may, for example, be an apparatus (such as USB memory) locally connected to the external device or a network interface for communication via a network. Further, the data communication method may be Wi-Fi (registered trademark) or USB communication.

The lipid measurement device 200 having the configuration described above carries out a lipid measurement process based on a program set in advance.

Figure 8 is a flowchart of the lipid measurement process.

The radiation unit 4 of the user device 400 radiates continuous light to a radiation position (step 201).

A first optical intensity detection unit 41 of the user device 400 detects the optical intensity in a first detection position, and a second optical intensity detection unit 42 detects the optical intensity in a second detection position (step S202).

The control unit 24 of the lipid measurement device 200 calculates the optical intensity difference or the optical intensity ratio between a first optical intensity in the first detection position and a second optical intensity in the second detection position and calculates the static parameter (scatter coefficient $\mu_s$') based on the optical intensity difference or the optical intensity ratio. Instead, the control unit 24 calculates the static parameter (average blood flow rate) from the optical intensity in the first detection position or the optical intensity in the second detection position (step 203).

The control unit 24 of the lipid measurement device 200 calculates the dynamic parameter, which is an index of the blood flow, from a temporal change in the static parameter (step 204). The control unit 4 may set the optical intensity measurement period to be 20 sec or shorter and calculate the dynamic parameter from the amount of change in the optical intensity within the measurement period.

The control unit 24 of the lipid measurement device 200 determines that the predetermined site of the living body irradiated with the light as a site appropriate for the lipid measurement based on the static parameter (and the dynamic parameter) (step 205). For example, the control unit 24 compares the data prepared in advance on the appropriate numerical range of each of the static parameter and the dynamic parameter with the calculated static parameter and the calculated dynamic parameter to evaluate whether or not the predetermined site is a site appropriate for the lipid measurement.

In the case where the static parameter is the scatter coefficient $\mu s'$ and the dynamic parameter is the variation coefficient CV, the control unit 24 determines that the predetermined site is a site appropriate for the lipid measurement when the scatter coefficient $\mu s'$ is greater than or equal to 0.4 but smaller than or equal to 0.53 and the variation coefficient CV is greater than or equal to 0.1% but smaller than or equal to 5.0%, preferably, the scatter coefficient $\mu s'$ is greater than or equal to 0.41 but smaller than or equal to 0.51 and the variation coefficient CV is greater than or equal to 0.2% but smaller than or equal to 1.5%, more preferably, the scatter coefficient $\mu s'$ is greater than or equal to 0.42 but smaller than or equal to 0.46 and the variation coefficient CV is greater than or equal to 0.5% but smaller than or equal to 1.0%.

In the case where the static parameter is the average blood flow rate and the dynamic parameter is the variation coefficient of the blood flow rate, the control unit 24 determines that the predetermined site is a site appropriate for the lipid measurement when the average blood flow rate is greater than or equal to 3.1 mL/min but smaller than or equal to 21.0 mL/min and the variation coefficient of the blood flow rate is greater than or equal to 5% but smaller than or equal to 50%, preferably, the average blood flow rate is greater than or equal to 5.1 mL/min but smaller than or equal to 15.0 mL/min and the variation coefficient of the blood flow rate is greater than or equal to 15% but smaller than or equal to 40%, more preferably, the average blood flow rate is greater than or equal to 5.1 mL/min but smaller than or equal to 13.0 mL/min and the variation coefficient of the blood flow rate is greater than or equal to 10% but smaller than or equal to 30%.

When the control unit 24 of the lipid measurement device 200 determines that the predetermined site is a site appropriate for the lipid measurement, the control unit 24 causes the notification unit 45 of the user device 400 to cause a buzzer to issue sound, a vibrator to vibrate, or a lamp to emit light (step 206).

[Example]

An example of the present invention will be described below, but the present invention is not limited to Example described below.

In the noninvasive lipid measurement, even when a single person is the measurement target, the person has a site where the amount of change in lipid concentration is measurable and a site where a site where the amount of change in lipid concentration is unmeasurable.

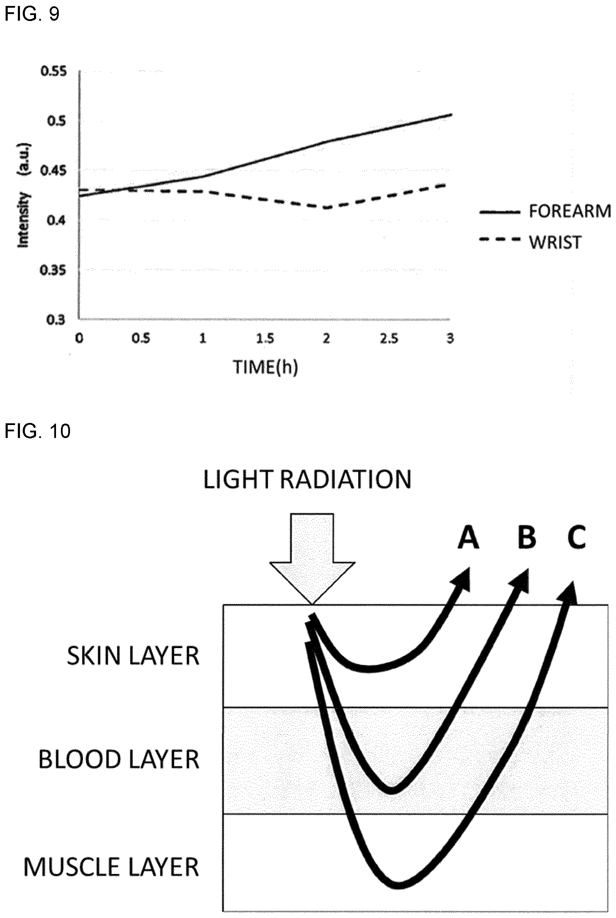

For example, a result of a lipid loading test shows that changes in lipid concentration can be measured in the forearm inner vein also on an individual basis but cannot be measured in a wrist (Figure 9).

Further, a result of measurement of the triceps brachii muscle, where a relatively large number of capillaries are present, shows that there are a subject who allows detection of changes in lipid concentration and a subject who does not allow measurement of changes in lipid concentration. That is, site differences are present in a single person, and individual differences are further present.

The reason why the differences are present is that the information obtained in the optical measurement contains all kinds of information, such as skin color, skin, and muscle contained in the skin layer, the blood layer, and the muscle layer present in the optical paths. The measurement is therefore affected by the depth of the blood layer, the amount of blood depending on the thickness of the blood vessel, and other factors (Figure 10).

Since the noninvasive lipid measurement is directed to lipid-containing blood as the measurement target, the present inventors have studied a method for efficiently extracting blood information. To acquire blood information, it is also conceivable to measure the amount of absorption of blood. In this case, however, the present inventors were concerned about the fact that using, for example, the wavelength at which hemoglobin is absorbed means using a wavelength different from the wavelength used in the lipid measurement and a resultant possible increase in size of the measurement device and determined to use a different approach in the present example.

The present inventors have instead focused on the motion of the blood. A result of the study conducted by the present inventors shows that the scattering at the skin or muscle does not greatly change in a short period, such as about 10 to 20 sec. It is readily expected that the result holds true at least in the measurement at the time of rest.

Among a variety of parameters, the motion of the blood changes in about 10 to 20 sec. For example, the scatter coefficients $\mu s'$ in a wrist and an upper arm inner vein in the hunger state are roughly equal to each other, and changes in lipid can be measured in the upper arm inner vein but cannot be measured in the wrist (Figure 9).

In the present example, an attempt to detect an optimum measurement site has been made by combining a static parameter containing influence of all substances that cause optical attenuation in the skin layer, the blood layer, and the muscle layer present in the optical paths, as shown in Figure 10, and the dynamic parameter representing the blood information. The static parameter is instantaneously measured data with no temporal axis considered.

As an index of the blood flow, comparison of the variation coefficients CV of the scatter coefficient $\mu$s' in 10-second measurement made at different sites shows that the variation coefficient CV in a wrist is 1.5% or higher, 30% in some cases. On the other hand, the variation coefficient CV is 1.5% or lower in the upper arm inner vein.

In the measurement made at a site other than the forearm veins, the variation coefficient CV was 1.5% or lower, but the scatter coefficient was small, so that it was difficult to detect changes in lipid.

The results described above show that the amount of blood present in the optical paths is important but there is an optimum amount of blood. When the variation coefficient CV is greater than 1.5%, too large an amount of blood is present in the lipid measurement, and the influence of dynamic scattering resulting from the blood blow is considered to cause scattering resulting from the lipid concentration to be relatively unlikely to be detected.

On the other hand, when the variation coefficient CV is too small, no blood flow is detected, and it is therefore difficult to perform the lipid measurement.

The same holds true for the scatter coefficient $\mu$s' in the hunger state. When the scatter coefficient $\mu$s' is too small, the amount of blood necessary for the blood measurement is not present, whereas when the scatter coefficient $\mu$s' is too large, the influence of the blood cells and skin color does not allow efficient lipid measurement. Since light attenuation depends on the two parameters, $\mu$s' and $\mu$a, as shown in Expression 3, it is speculated that the effect of the scatter coefficient is hindered when $\mu$a>>$\mu$s'. The coefficient $\mu$a may therefore be measured, for example, in another approach.

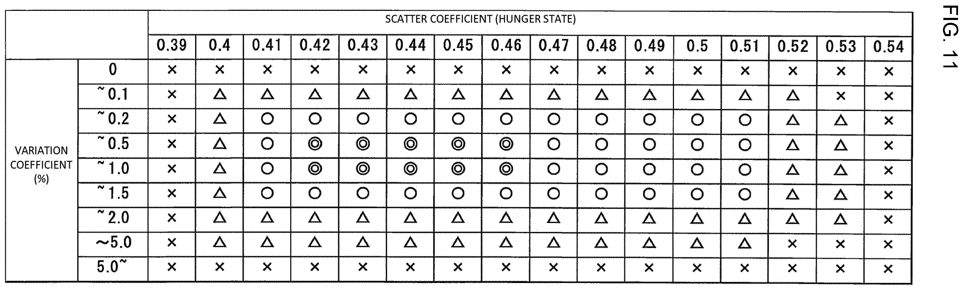

A test has been performed on three subjects to verify the ranges of the scatter coefficient $\mu$s' and the variation coefficient CV (Figure 11).

As a result, the present inventors have found that a change in lipid concentration can be measured when a measurement site satisfies the following conditions at the same time. The ranges of the scatter coefficient $\mu$s' and the variation coefficient CV that allow measurement of a change in lipid concentration are as follows: The scatter coefficient $\mu$s' is greater than or equal to 0.4 but smaller than or equal to 0.53 and the variation coefficient CV is greater than or equal to 0.1% but smaller than or equal to 5.0% (regions labeled with "triangle" in Figure 11), preferably, the scatter coefficient $\mu$s' is greater than or equal to 0.41 but smaller than or equal to 0.51 and the variation coefficient CV is greater than or equal to 0.2% but smaller than or equal to 1.5% (regions labeled with "single circle" in Figure 11), further preferably, the scatter coefficient $\mu$s' is greater than or equal to 0.42 but smaller than or equal to 0.46 and the variation coefficient CV is greater than or equal to 0.5% but smaller than or equal to 1.0% (regions labeled with "double circle" in Figure 11).

Under the conditions described above, about 95% of the entire range shown in Figure 11 is the range where a change in lipid concentration is measurable, and the range can be widened in typical applications and can be narrowed in medical applications to increase the accuracy of the measurement.

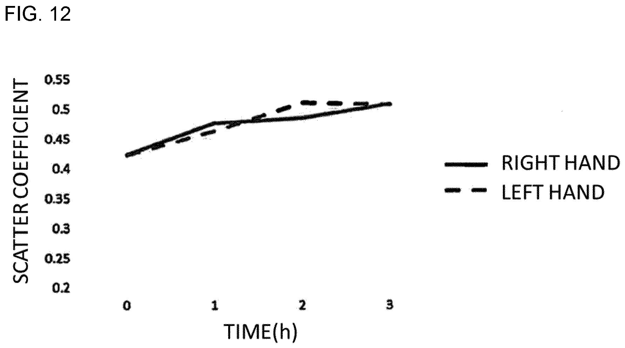

Figure 12 shows results of the measurement made on arbitrary sites of the right and left hands where the conditions described above are satisfied. As shown in Figure 12, measurement of a site where the scatter coefficient $\mu$s' and the variation coefficient CV are close to each other allows a site appropriate for the lipid measurement to be found. It is speculated that the CV measurement depends on the detection sensitivity, the sampling rate, the bit depth, and other factors of device performance, and it is therefore desirable to determine an optimum CV value on a device specification basis.

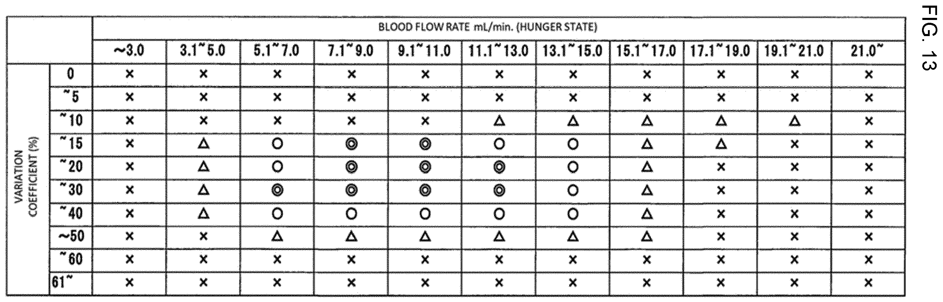

Similarly, Figure 13 shows a case where a laser blood flow meter is used. The ranges of the average blood flow rate and the variation coefficient of the blood flow rate that allow measurement of a change in lipid concentration are as follows: The average blood flow rate is greater than or equal to 3.1 mL/min but smaller than or equal to 21.0 mL/min and the variation coefficient of the blood flow rate is greater than or equal to 5% but smaller than or equal to 50% (regions labeled with "triangle" in Figure 13), preferably, the average blood flow rate is greater than or equal to 5.1 mL/min but smaller than or equal to 15.0 mL/min and the variation coefficient of the blood flow rate is greater than or equal to 15% but smaller than or equal to 40% (regions labeled with "single circle" in Figure 13), further preferably, the average blood flow rate is greater than or equal to 5.1 mL/min but smaller than or equal to 13.0 mL/min and the variation coefficient of the blood flow rate is greater than or equal to 10% but smaller than or equal to 30% (regions labeled with "double circle" in Figure 13).

It is, however, noted that when a different approach in terms of principle or a different device in terms of accuracy is used, values different from the values described above need to be set.

The measurement conditions can be determined by performing the measurement for about 10 sec, and the device can notify the user in the form of sound issued from a buzzer, vibration, light emitted from a lamp, or any other signal.

The period of 10 sec is determined in consideration of a period that allows a person to recognize that appropriate measurement conditions have been achieved and is therefore not necessarily required to determine the measurement conditions. Instead, the device may evaluate the measurement conditions in about 0.1 to 1.0 sec, and the measurer may check for about 3 to 10 sec that the values have become stable.

The technology described above can also be used to search for the position of a vein or an artery.

The light receiver, when it is formed, for example, of an array of light receiving elements, a CCD camera, or a CMOS camera, can acquire two-dimensional information, and a program can be used to automatically detect an optimum position where the conditions 1 and 2 described above are satisfied.

In the present technology, in which the lipid measurement can be performed based on relative optical attenuation of the intensity of the light radiated to a light receiving site, the light source as part of the device configuration is not limited to an LED or a LD but may be sunlight or room light. In this case, light toward a measurement site may be blocked, and the measurement site may be irradiated with light, for example, through an optical fiber, or a pinhole may be formed in the light blocker. Also in this case, the positions of a vein or an artery can be estimated based on the optical attenuation.

An embodiment has been described above, and the embodiment is presented by way of example and is not intended to limit the scope of the invention. The novel embodiment can be implemented in a variety of other forms, and a variety of omissions, replacements, and changes can be made to the embodiment to the extent that they do not depart from the substance of the present invention. The embodiment and variations thereof are encompassed in not only the scope and substance of the invention but the invention set forth in the claims and the scope equivalent thereto.

Reference Signs List

1:     Lipid measurement device
2:     Radiation unit
3:     Optical intensity detection unit
4:     Control unit
5:     Notification unit

**Claims**

1.   A lipid measurement device comprising:

a radiation unit that radiates light having a predetermined optical intensity to a predetermined site of a living body from a point outside the living body toward an interior of the living body;
an optical intensity detection unit that is spaced apart by a predetermined gap or continuously from a position irradiated with the light from the radiation unit and detects optical intensity of light emitted from the living body in at least one position; and
a control unit that calculates a static parameter in the living body based on the optical intensity detected by the optical intensity detection unit, calculates a dynamic parameter that is an index of motion of blood based on a temporal change in the static parameter, and

determines a living body site appropriate for lipid measurement based on the static parameter and the dynamic parameter.

2.   The lipid measurement device according to claim 1, wherein the living body site appropriate for lipid measurement is a skin layer that is a surface layer where a vein or a capillary is present.

3.   The lipid measurement device according to claim 1 or 2, wherein the control unit sets a measurement period for which the optical intensity is measured at 20 sec or shorter and calculates the dynamic parameter based on an amount of change in the optical intensity in the measurement period.

4.   The lipid measurement device according to any of claims 1 to 3,
wherein the static parameter is a scatter coefficient, and
the control unit calculates an optical scatter coefficient in the living body based on a ratio or a difference among optical intensities detected in a plurality of positions by the optical intensity detection unit.

5.   The lipid measurement device according to claim 4 wherein the dynamic parameter is a variation coefficient, and
the control unit calculates the variation coefficient based on a change in the scatter coefficient over a predetermined period.

6.   The lipid measurement device according to claim 5, wherein the control unit determines that the predetermined site is a site appropriate for the lipid measurement when the scatter coefficient is greater than or equal to 0.40 but smaller than or equal to 0.53 and the variation coefficient is greater than or equal to 0.1% but smaller than or equal to 5.0%.

7.   The lipid measurement device according to claim 5, wherein the control unit determines that the predetermined site is a site appropriate for the lipid measurement when the scatter coefficient is greater than or equal to 0.41 but smaller than or equal to 0.51 and the variation coefficient is greater than or equal to 0.2% but smaller than or equal to 1.5%.

8.   The lipid measurement device according to claim 5, wherein the control unit determines that the predetermined site is a site appropriate for the lipid measurement when the scatter coefficient is greater than or equal to 0.42 but smaller than or equal to 0.46 and the variation coefficient is greater than or equal to 0.5% but smaller than or equal to 1.0%.

9.   The lipid measurement device according to any of claims 5 to 8, wherein the predetermined period is longer than or equal to 10 msec but shorter than or equal to 20 sec.

10. The lipid measurement device according to any of claims 1 to 3,
wherein the static parameter is an average blood flow rate, and
the control unit calculates the average blood flow rate in the living body based on the optical intensity detected by the optical intensity detection unit.

11. The lipid measurement device according to claim 10,
wherein the dynamic parameter is a variation coefficient of the blood flow rate, and
the control unit calculates the variation coefficient of the blood flow rate based on a temporal change in the average blood flow rate.

12. The lipid measurement device according to claim 11,
wherein the control unit determines that the predetermined site is a site appropriate for the lipid measurement when the average blood flow rate is greater than or equal to 3.1 mL/min but smaller than or equal to 21.0 mL/min and the variation coefficient of the blood flow rate is greater than or equal to 5% but smaller than or equal to 50%.

13. The lipid measurement device according to claim 11,
wherein the control unit determines that the predetermined site is a site appropriate for the lipid measurement when the average blood flow rate is greater than or equal to 5.1 mL/min but smaller than or equal to 15.0 mL/min and the variation coefficient of the blood flow rate is greater than or equal to 15% but smaller than or equal to 40%.

14. The lipid measurement device according to claim 11,
wherein the control unit determines that the predetermined site is a site appropriate for the lipid measurement when the average blood flow rate is greater than or equal to 5.1 mL/min but smaller than or equal to 13.0 mL/min and the variation coefficient of the blood flow rate is greater than or equal to 10% but smaller than or equal to 30%.

15. The lipid measurement device according to any of claims 1 to 3, wherein the dynamic parameter is an index for analyzing the motion of the blood by using a standard deviation, Brownian motion, an autocorrelation function, frequency analysis, speckles, Doppler shift, Reynold's number, blood flow rate, an amount of blood, or pulsation width to measure the motion of the blood.

16. A lipid measurement method that causes a computer of a lipid measurement device including a radiation unit that radiates light having a predetermined optical intensity to a predetermined site of a living body from a point outside the living body toward an interior of the living body and an optical intensity detection unit that is spaced apart by a predetermined gap or continuously from a position irradiated with the light from the radiation unit and detects optical intensity of light emitted from the living body in at least one position to carry out the processes of:

calculating a static parameter in the living body based on the optical intensity detected by the optical intensity detection unit;
calculating a dynamic parameter that is an index of motion of blood based on a temporal change in the static parameter; and
determining a living body site appropriate for lipid measurement based on the static parameter and the dynamic parameter.

17. A lipid measurement device connected to a user device including
a radiation unit that radiates light having a predetermined optical intensity to a predetermined site of a living body from a point outside the living body toward an interior of the living body, and
an optical intensity detection unit that is spaced apart by a predetermined gap or continuously from a position irradiated with the light from the radiation unit and detects optical intensity of light emitted from the living body in at least one position,
the lipid measurement device comprising a control unit that
calculates a static parameter in the living body based on the optical intensity detected by the optical intensity detection unit,
calculates a dynamic parameter that is an index of motion of blood based on a temporal change in the static parameter, and
determines a living body site appropriate for lipid measurement based on the static parameter and the dynamic parameter.

FIG. 1

FIG. 2

FIG. 3

FIG. 4

FIG. 5

S101 ——————— | RADIATE LIGHT |

S102 ——————— | DETECT OPTICAL INTENSITY |

S103 ——————— | CALCULATE STATIC PARAMETER |

S104 ——————— | CALCULATE DYNAMIC PARAMETER |

S105 ——————— | DETERMINE MEASUREMENT SITE |

S106 ——————— | PERFORM NOTIFICATION |

FIG. 6

FIG. 7

FIG. 8

S201 ── RADIATION STEP

S202 ── DETECT OPTICAL INTENSITY

S203 ── CALCULATE STATIC PARAMETER

S204 ── CALCULATE DYNAMIC PARAMETER

S205 ── DETERMINE MEASUREMENT SITE

S206 ── PERFORM NOTIFICATION

FIG. 9

FIG. 10

## FIG. 11

| VARIATION COEFFICIENT (%) | SCATTER COEFFICIENT (HUNGER STATE) | | | | | | | | | | | | | | | |
|---|---|---|---|---|---|---|---|---|---|---|---|---|---|---|---|---|
| | 0.39 | 0.4 | 0.41 | 0.42 | 0.43 | 0.44 | 0.45 | 0.46 | 0.47 | 0.48 | 0.49 | 0.5 | 0.51 | 0.52 | 0.53 | 0.54 |
| 0 | × | × | × | × | × | × | × | × | × | × | × | × | × | × | × | × |
| ~0.1 | × | △ | △ | △ | △ | △ | △ | △ | △ | △ | △ | △ | △ | △ | × | × |
| ~0.2 | × | △ | ○ | ○ | ○ | ○ | ○ | ○ | ○ | ○ | ○ | ○ | ○ | △ | △ | × |
| ~0.5 | × | △ | ○ | ◎ | ◎ | ◎ | ◎ | ◎ | ○ | ○ | ○ | ○ | ○ | △ | △ | × |
| ~1.0 | × | △ | ○ | ◎ | ◎ | ◎ | ◎ | ◎ | ○ | ○ | ○ | ○ | ○ | △ | △ | × |
| ~1.5 | × | △ | ○ | ○ | ○ | ○ | ○ | ○ | ○ | ○ | ○ | ○ | ○ | △ | △ | × |
| ~2.0 | × | △ | △ | △ | △ | △ | △ | △ | △ | △ | △ | △ | △ | △ | △ | × |
| ~5.0 | × | △ | △ | △ | △ | △ | △ | △ | △ | △ | △ | △ | △ | △ | × | × |
| 5.0~ | × | × | × | × | × | × | × | × | × | × | × | × | × | × | × | × |

FIG. 12

FIG. 13

BLOOD FLOW RATE mL/min. (HUNGER STATE)

| VARIATION COEFFICIENT (%) | ~3.0 | 3.1~5.0 | 5.1~7.0 | 7.1~9.0 | 9.1~11.0 | 11.1~13.0 | 13.1~15.0 | 15.1~17.0 | 17.1~19.0 | 19.1~21.0 | 21.0~ |
|---|---|---|---|---|---|---|---|---|---|---|---|
| 0 | × | × | × | × | × | × | × | × | × | × | × |
| ~5 | × | × | × | × | × | × | × | × | × | × | × |
| ~10 | × | × | × | × | × | △ | △ | △ | △ | △ | × |
| ~15 | × | △ | ○ | ◎ | ◎ | ○ | ○ | △ | △ | × | × |
| ~20 | × | △ | ○ | ◎ | ◎ | ◎ | ○ | △ | × | × | × |
| ~30 | × | △ | ◎ | ◎ | ◎ | ◎ | ○ | △ | × | × | × |
| ~40 | × | △ | ○ | ○ | ○ | ○ | ○ | △ | × | × | × |
| ~50 | × | × | △ | △ | △ | △ | △ | △ | × | × | × |
| ~60 | × | × | × | × | × | × | × | × | × | × | × |
| 61~ | × | × | × | × | × | × | × | × | × | × | × |

<div align="center">

**INTERNATIONAL SEARCH REPORT**

</div>

| | International application No. |
|---|---|
| | PCT/JP2017/045667 |

| A. CLASSIFICATION OF SUBJECT MATTER |
|---|
| Int. Cl. A61B5/1455(2006.01)i |

According to International Patent Classification (IPC) or to both national classification and IPC

| B. FIELDS SEARCHED |
|---|
| Minimum documentation searched (classification system followed by classification symbols) |
| Int. Cl. A61B5/1455 |

Documentation searched other than minimum documentation to the extent that such documents are included in the fields searched

```
Published examined utility model applications of Japan      1922-1996
Published unexamined utility model applications of Japan    1971-2018
Registered utility model specifications of Japan            1996-2018
Published registered utility model applications of Japan    1994-2018
```

Electronic data base consulted during the international search (name of data base and, where practicable, search terms used)

| C. DOCUMENTS CONSIDERED TO BE RELEVANT |
|---|

| Category* | Citation of document, with indication, where appropriate, of the relevant passages | Relevant to claim No. |
|---|---|---|
| Y<br>A | JP 2017-532541 A (RSP SYSTEMS A/S) 02 November 2017, paragraphs [0096], [0138], [0139] & US 2017/0273564 A1, paragraphs [0096], [0149], [0150] | 1-3, 10-17<br>4-9 |
| Y | JP 2010-264126 A (KONICA MINOLTA SENSING, INC.) 25 November 2010, paragraphs [0056]-[0060] (Family: none) | 1-3, 10-17 |
| Y | JP 2015-167790 A (TOSHIBA CORP.) 28 September 2015, paragraph [0018] & US 2015/0250395 A1, paragraph [0018] | 10-14 |

| ☐ Further documents are listed in the continuation of Box C. | ☐ See patent family annex. |
|---|---|

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 15.03.2018 | 27.03.2018 |

| Name and mailing address of the ISA/<br>Japan Patent Office<br>3-4-3, Kasumigaseki, Chiyoda-ku,<br>Tokyo 100-8915, Japan | Authorized officer |
|---|---|
| | Telephone No. |

Form PCT/ISA/210 (second sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2014087825 A